(19) Europäisches Patentamt
European Patent Office
Office européen des brevets

(11) Publication number: 0 363 532
A1

(12) **EUROPEAN PATENT APPLICATION**

(21) Application number: 88309456.7

(22) Date of filing: 10.10.88

(51) Int. Cl.5: C07D 241/12 , B01J 31/00 , B01J 23/00

(43) Date of publication of application: 18.04.90 Bulletin 90/16

(84) Designated Contracting States: DE GB

(71) Applicant: TEXACO DEVELOPMENT CORPORATION
2000 Westchester Avenue
White Plains, New York 10650(US)

(72) Inventor: Su, Wei-Yang
3543 Greystone Dr. No. 2118
Austin Texas 78731(US)
Inventor: Knifton, John Frederick
10900 Catskill Trail
Austin Texas 78750(US)

(74) Representative: Harrison, Michael Robert et al
Urquhart-Dykes & Lord 91 Wimpole Street
London W1M 8AH(GB)

# (54) Process for preparing pyrazines from hydroxyamines.

(57) This invention relates to the reaction of hydroxyamines to form pyrazines by a process comprising reacting said hydroxyamine in the presence of a catalyst which may be a transition metal catalyst optionally containing an alkaline earth metal oxide, or alternatively the catalyst may be a rhodium, ruthenium or palladium-containing compound and a phosphine-containing compound.

EP 0 363 532 A1

## PROCESS FOR PREPARING PYRAZINES FROM HYDROXYAMINES

This invention concerns a process for the preparation of pyrazines. More particularly this invention relates to the preparation of pyrazines under mild reaction conditions from hydroxyamines in the presence of a homogeneous ruthenium, rhodium or palladium-containing compound and a phosphine compound, or in the presence of a heterogeneous transition metal catalyst optionally containing an alkaline earth metal oxide.

Previously, the preparation of pyrazine and its derivatives has involved the dehydrogenation of the corresponding piperazines, but the method suffers from the disadvantage that the two classes of chemicals are often difficult to separate.

Pyrazines have also been prepared from diols, for example Japanese Patent No. 79,132,588 discloses the preparation of pyrazines by treating diols with diamines in the gas phase in the presence of compounds such as Zn, Mg, Ce, Mn, Fe, Pd, Pb, P or B. In one example 2,3-butanediol and 1,2-diaminopropane were reacted in the presence of a ZnO catalyst containing $PdSO_4$ at 470-480 °C to give a 70% pyrazine product with 100% conversion of the starting materials.

In another Japanese reference, Japanese Patent No. 80,122,769, a method is disclosed for preparing pyrazines from the hydroxydiamine, N-(2-hydroxyethyl)ethylenediamine using a $Cr_2O_3$-CuO catalyst at a temperature from about 265 °C to 300 °C. The conversion was about 82% and the yield of pyrazine was about 78%.

Pyrazines have also been prepared from diols plus a diamine through contact in the gas phase in the presence of a zinc-containing catalyst. Such a procedure is disclosed in U. S. Patent No. 4,097,478.

To our knowledge, the only disclosure of a procedure for making pyrazines from hydroxyamines comes from a recent Japanese Patent Application, 60258-168A, which describes subjecting alkanolamines to gaseous reaction conditions in the presence of a zinc-containing catalyst. High temperatures of 300-500 °C are preferred for this synthesis with this class of catalyst.

The pyrazine products of these syntheses described supra are useful as intermediates for perfumes, pharmaceuticals and agrochemicals. 2,5-Dimethylpyrazine, for example, is useful in cosmetics, flavorings and polymer applications.

It would be a great advantage in the art if pyrazines could be prepared from hydroxyamines under mild reaction conditions. It would also be an advantage if the catalyst system which allowed good conversion and yield also enabled the product to be separated efficiently.

It has now been discovered that pyrazines can be prepared by a process which comprises cyclocondensation of a hydroxyamine in the presence of a catalyst comprising a rhodium, ruthenium or palladium-containing compound and a phosphine containing compound or in the presence of one or more transition metals selected from copper, nickel, chromium and palladium, the reaction being carried out at elevated temperature and at atmospheric pressure or greater.

The pyrazines can be represented by the structural formula:

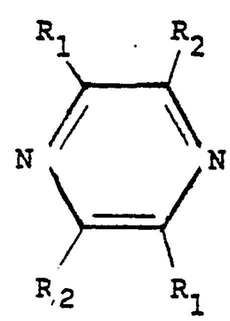

Suitable hydroxyamine reactants for use with a homogeneous catalyst have the structural formula:

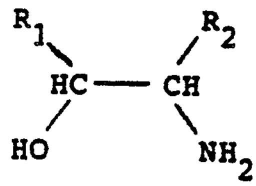

wherein $R_1$ is a hydrocarbon group having one to about 6 carbon atoms, such as an alkyl, alkenyl or cycloalkyl group and $R_2$ hydrogen or of the class of $R_1$.

More specifically, suitable hyroxyamine reactants for use with the heterogeneous transition metal catalyst comprise $R_1$ being a hydrocarbon group having 1 to about 10 carbon atoms selected from alkyl, aryl, aralkyl, alkaryl, alkenyl or cycloalkyl group and $R_2$ is hydrogen or of the class of $R_1$. Suitable hydroxyamines for synthesis of pyrazines include Beta-hydroxyamines or 1,2-hydroxyamines.

The pyrazines which are a product of this invention have several uses. For example, 2,5-dimethylpyrazine, the main product in the Examples, has market in cosmetics, flavorings and the polymer industries.

In the narrower and more preferred practice of this invention, pyrazines are prepared from a hydroxyamine by a process comprising:

A 1) Contacting said $\beta$-hydroxyamine with a catalyst system comprising a homogeneous transition metal compound selected from the group consisting of ruthenium, rhodium or palladium, plus a phosphine-containing compound,

2) heating said reacting mixture to a temperature of at least 100°C under atmospheric pressure or greater, and

3) separating said pyrazine products contained therein.

B 1) Contacting said hydroxyamine with a catalyst system comprising a heterogeneous transition metal catalyst containing an alkaline earth metal oxide,

2) heating said reacting mixture to a temperature of at least 50°C under atmospheric pressure or greater, and

3) separating said pyrazine products contained therein.

The reaction of hydroxyamines to form pyrazines can be represented by the following equation:

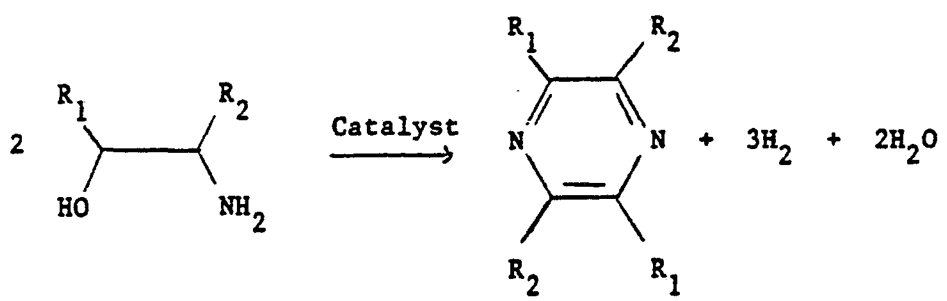


(Eq. I)

## A) EXAMPLES FOR THE USE OF A HOMOGENEOUS CATALYST

In general, the components of the reaction mixture, including the hydroxyamine, transition metal compound, phosphine compound and optional solvent may be added in any sequence as long as good agitation is employed to provide a good dispersion or homogeneous reaction mixture. For example, the following represent some variations insofar as the addition of catalyst components, solvents and hydroxyamine that can be made without departing from the inventive process. These modifications include:

1. The catalyst may be preformed and added to the solvent prior to addition of the hydroxyamine;

2. Preferably, to minimize stability problems with the catalyst, the catalyst is best formed in situ, usually by mixing the solvent and hydroxyamine followed by the addition of the transition metal compound and phosphorous-containing compound to form the reaction mixture.

3. After using either variation 1 or 2 the catalyst-containing reaction mixture is heated until the product is formed.

In order to present the inventive concept in the greatest possible detail to promote its understanding, the following supplementary disclosure is submitted. The basic invention improved upon here is practised as follows:

The reactant used in the process of this invention comprises a hydroxyamine.

The catalyst comprises a transition metal, phosphine and solvent.

Catalysts which are suitable in the practice of this invention contain transition metals of the group consisting of ruthenium, rhodium or palladium. The transition metal can be chosen from a wide variety of organic or inorganic compounds, complexes, etc. It is only necessary that the catalyst precursor actually employed contain said metal in any of its ionic states. The actual catalytically active species is then believed to comprise ruthenium, rhodium or palladium and phosphine in complex combination with a hydroxyamine in a solvent. The most effective catalyst is believed to be achieved where a ruthenium, rhodium or palladium salt of a mineral acid is mixed with a phosphine compound in a solvent under reaction conditions.

The ruthenium catalyst precursors may take many different forms. For instance, the ruthenium may be added to the reaction mixture in an oxide form, as in the case of, for example, ruthenium(IV) oxide hydrate. Alternatively, it may be added as the salt of a mineral acid, as in the case of ruthenium(III) chloride hydrate, ruthenium(III) bromide, ruthenium(III) iodide, tricarbonyl ruthenium(II) iodide, anhydrous ruthenium-(III) chloride and ruthenium nitrate, or as the salt of a suitable organic carboxylic acid, such as, for example, ruthenium(III) acetate, ruthenium naphthenate, ruthenium valerate and ruthenium complexes with carbonyl-containing ligands, such as ruthenium(III) acetylacetonate. The ruthenium may also be added to the reaction zone as a carbonyl or hydridocarbonyl derivative. Here, suitable examples include triruthenium dodecacarbonyl and other hydridocarbonyls such as $H_2Ru_4(CO)_{13}$ and $H_4Ru_4(CO)_{12}$, and substituted carbonyl species such as the tricarbonylruthenium(II) chloride dimer, $[Ru(CO)_3Cl_2]_2$.

The rhodium-catalyst precursors may also take many different forms. For instance, the rhodium may be added to the reaction mixture in an oxide form, as in the case of, for example, rhodium (III)- oxide. Alternatively, it may be added as the salt of a mineral acid, as in the case of rhodium(III) chloride, rhodium bromide and rhodium(III) nitrate, or as the salt of a suitable organic carboxylic acid, such as, for example, rhodium(II) acetate dimer, as well as rhodium complexes with carbonyl-containing ligands, such as rhodium-(III) acetylacetonate. The rhodium may also be added to the reaction zone as a carbonyl or hydrido carbonyl derivative. Here, suitable examples include tetrarhodium dodecacarbonyl and hexarhodium hexadecacarbonyl.

The palladium catalyst precursor may take several different forms. For instance, the palladium may be added as the salt of a mineral acid, as in the case of palladium(II) chloride and palladium(II) bromide, or as the salt of a suitable organic carboxylic acid, such as, for example, palladium(II) acetate, as well as palladium complexes with carbonyl-containing ligands such as palladium(II) acetylacetonate. The palladium may also be added to the reaction zone as a carbonyl or carbonyl derivative, or as a nitrile complex, as in the case of dichloro(benzonitrile)palladium(II).

The catalyst compounds are preferably employed with a tertiary phosphine. Suitable tertiary phosphine components used in the preferred catalyst formulation may contain one or more trivalent phosphorous atoms per molecule, wherein the phosphorus is bonded to alkyl, aryl, alkaryl and aralkyl reactants or mixtures thereof. Specific examples of such tertiary phosphines include tri-n-butylphosphine, tri-sec-butylphosphine, trimethylphosphine, triethylphosphine, tri-c-hexylphosphine, triphenylphosphine, triptolylphosphine, benzyldiphenylphosphine, 1,2-bis(diphenylphosphino)ethane,tri-p-methoxyphenylphosphine, 1,3bis(diphenylphosphino)propane among others.

In the preferred embodiment of this invention the catalyst comprises a phosphine compound used in conjunction with the rhodium, ruthenium or palladium. The preferred catalyst includes a transition metal salt of a mineral acid and a trialkylphosphine. These components may be added as a preformed complex or complexes, or they may be added separately to the reaction zone. The preferred combinations include: ruthenium(III) chloride plus tri-n-butylphosphine, $RhCl_3$-$Ph_2P(CH_2)_2PPh_2$, $RuCl_3$-$Ph_2P(CH_2)_3PPh_2$, dichlorotris(triphenylphosphine)ruthenium(II), dichlorodicarbonylbis(triphenylphosphine)ruthenium(II) and hydrido(acetato)tris(triphenylphosphine)ruthenium(II). Preferred rhodium-phosphine combinations include $RhCl_3Ph_2P(CH_2)_3PPh_2$, hydridocarbonyltris(triphenylphosphine)rhodium(I), chlorotris(triphenylphosphine)-rhodium(I), chlorocarbonylbis(triphenylphosphine)rhodium(I), and $RhCl_3$-$PBu_3$. Preferred palladium-phosphine combinations include dichlorobis(triphenylphosphine)palladium(II) and tetrakis-(triphenylphosphine)palladium(O).

As mentioned above the preferred reactants are hydroxyamines. The hydroxyamines which will work in the process can be represented by the following structural formula:

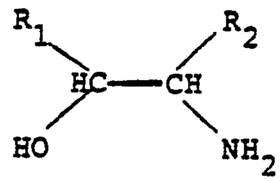

wherein $R_1$ is a hydrocarbon group having one to about 6 carbon atoms, such as an alkyl, alkenyl or cycloalkenyl group and $R_2$ is hydrogen or of the class of $R_1$.

Examples of suitable β-hydroxyamines include, but are not limited, to ethanolamine, 1-amino-2-propanol, 1-amino-2-butanol, 1-amino-2-hexanol and 2-amino-3-butanol. Good results were obtained using 3-amino-2-propanol.

The novel reaction is run most conveniently in the presence of a solvent. The solvent useful in the process of this invention is an oxygenated hydrocarbon, i.e., a compound composed only of carbon, hydrogen and oxygen and one in which the only oxygen atoms present are in ether groups. Generally the oxygenated hydrocarbon will contain 3 to 14 carbon atoms and preferably a maximum of 7 oxygen atoms. The solvent must be substantially inert under reaction conditions.

Ethers which may be utilized as solvents include cyclic, acyclic and heterocyclic ethers, as illustrated by 1,4-dioxane and 1,3-dioxane. Other suitable ether solvents include isopropyl propyl ether, diethylene glycol dibutyl ether, dibutyl ether, ethyl butyl ether, diphenyl ether, heptyl phenyl ether, anisole, tetrahydrofuran, tetraethylene glycol dimethyl ether (tetraglyme) etc.

The preferred solvent in the reaction to produce pyrazines is p-dioxane.

The quantity of transition metal compound, phosphine compound and solvent employed in the instant invention is not critical and may vary over a wide range. In general, the novel process is desirably conducted in the presence of a catalytically effective quantity of active transition metal species, phosphine compound and solvent which gives the desired product in reasonable yield. The reaction proceeds when employing as little as about 1 x $10^{-6}$ weight percent and even lesser amounts of transition metal.

The upper concentration is dictated by a variety of factors including catalyst cost, partial pressure of carbon monoxide and hydrogen, operating temperature, etc. A transition metal concentration of from about 0.0001 to about 10 weight percent in conjunction with a phosphine concentration of from about 0.001 to about 10 weight percent and a solvent concentration of from zero to about 70% based on the total weight of the reaction mixture, is desirable in the practice of this invention.

The temperature range which can usefully be employed in these syntheses is a variable dependent upon other experimental factors, including the pressure, the concentration and the choice of the particular species of transition metal catalyst, among other things. The range of operability is from about 50° to 300° C. A narrow range of 150°-250° C represents the preferred temperature range.

Pressures of atmospheric or greater lead to substantial yields of pyrazine products by the process of this invention. A preferred operating range is above 50 psi. The most preferred range is from 100-400 psi, but pressures greater than 400 psi can be used.

The novel process of this invention can be conducted in a batch, semi-continuous or continuous fashion and said material may be recovered by methods well known in the art, such as distillation, fractionation, extraction and the like.

The major products of this syntheses are pyrazines, including, but not limited to 2,5-dimethyl-3-propylpyrazine, 2,5-dimethyl-3-ethylpyrazine, 2,3,5-trimethylpyrazine and 2,5-dimethylpyrazine The principal by-products of these preparations are piperazines and their derivatives.

Yield, as defined herein, represents the efficiency in catalyzing the desired reaction relative to other undesired reactions. In this instance synthesis of pyrazines is the desired conversion. Yield is expressed as a molar percentile and is calculated by determining the molar amount of, for example, 2,5-dimethylpyrazine formed, divided by the molar amount of hydroxyamine charged and multiplying the quotient.

The products have been identified in this work by one or more of the following analytical procedures, viz, gas-liquid phase chromatography (glc), infrared (ir), mass spectrometry, proton nuclear magnetic resonance (H′-nmr) and elemental analyses, or a combination of these techniques. All temperatures are in degrees centigrade and all pressures are in pounds per square inch gauge (psig).

Having described the inventive process, the following examples are submitted to supply specific and illustrative embodiments.

## SPECIFIC EXAMPLE 1

A 300-ml stirred autoclave with Pyrex liner was charged with a mixture of 1-amino-2-propanol (9.1g, 0.12 mol), tributylphosphine (0.8 ml), ruthenium trichloride (0. 265g), and p-dioxane (20 ml). The reactor was sealed and purged of air. The reactor was heated to 180°C and held for 5 hours. During this process, the pressure within the reactor reached 250 psi. The reaction mix was then allowed to cool to room temperature, and the gas formed during the reaction was vented. The product liquid (29.89) was recovered and analyzed.

A 52.8% yield of 2,5-dimethylpyrazine and 13.3% yield of 2,5-dimethylpiperazine were obtained, with 99% conversion of the 1-amino-2-propanol charged.

## SPECIFIC EXAMPLES 2 - 7

A variety of catalysts and conditions were tested for the cyclocondensation of 1-amino-2-propanol using the method of Example 1. The results are shown in Table III.

TABLE III

| Exp. | Catalyst | Ratio of Catalyst to Hydroxyamine w/w | Temp., °C | Time, Hr | Max Press., psi | Conversion % | Yield, % | |
|---|---|---|---|---|---|---|---|---|
| | | | | | | | 2,5-dimethylpyrazine | 2,5-dimethylpiperazine |
| 1. | $RuCl_3$-$Bu_3P$ | 0.029 | 180 | 5 | 150 | 99.3 | 52.8 | 13.3 |
| 2. | $RuCl_3$-$Bu_3P$ | 0.018 | 165 | 12 | 350 | 51.2 | 29.1 | 2.3 |
| 3. | $RuCl_2(PPH_3)_3$ | 0.01 | 180 | 12 | 200 | 16.3 | 10.9 | 0.9 |
| 4. | $RuCl_3$-$Ph_2P$-$(CH_2)_3PPh_2$ | 0.016 | 180 | 11 | 300 | 69.6 | 12.6 | 5.7 |
| 5. | $RhCl_3$-$Ph_2P$-$(CH_2)_3PPh_2$ | $6.25 \times 10^{-4}$ | 180 | 9 | 300 | 9.1 | 6.0 | 0.3 |
| 6. | $PdCl_2(PPh_3)_2$ | 0.01 | 180 | 10 | 100 | 6.1 | 2.9 | 0.5 |
| 7. | $Hrh(CO)(PPh_3)_3$ | 0.005 | 180 | 12 | 350 | 65.9 | 23.3 | 10.3 |

SPECIFIC EXAMPLE 8

The 1-amino-2-butanol (43g, 0.48 mol), ruthenium trichloride (0.52g) and tributylphosphine (1.6 ml) were subjected to a reaction as described in Example 1 above except that the solvent was tetraglyme (30 ml). A 23% yield of 2.5-diethylpyrazine and a 17% yield of 2,5-diethylpiperazine were obtained with 61% conversion of 1-amino-2-butanol.

## B) EXAMPLES FOR THE USE OF A HETEROGENEOUS TRANSITION METAL CATALYST

Generally, the reaction of the hydroxyamine may be carried out batchwise in an autoclave or continuously in, for example, a tubular plug-flow reactor. Although the order of addition of reactants and catalyst components may vary, a general procedure is to charge the transition metal containing an alkaline earth metal oxide to the reactor in the form of a powder, extrudate or pellet of a transition metal containing the oxide. Commercially prepared catalyst may be used. For example, Harshaw Cu-0401 and Cu-0402 contain not only the transition metal copper, but also barium oxide. Although the order of addition of reactant and catalyst components may vary, a general batch synthesis procedure is to charge the heterogeneous transition metal catalyst containing an alkaline earth metal oxide and hydroxyamine to an appropriate reactor, and increase the pressure and temperature to a desired level for an appropriate period to produce the desired pyrazine product.

As mentioned hereinabove a transition metal catalyst compound which contains an alkaline earth metal oxide is employed in the process of the invention and provides a catalyst system which demonstrates good yields of pyrazines from hydroxyamines with low yields of piperazine and allows for ease of separation of the desired pyrazine product, a feature which is commercially attractive and desired in the art. Said transition metal catalyst may contain as the transition metal component one or more of the following elements: copper, nickel, palladium and chromium. The preferred catalysts also contain an alkaline earth metal oxide selected from the group including barium oxide, strontium oxide, calcium oxide and magnesium oxide. The alkaline earth metal oxide may comprise up to 30 percent by weight of the total catalyst composition. Active catalyst compositions may also contain significant quantities of cadmium.

In order to present the inventive concept in the greatest possible detail to promote its understanding, the following supplementary disclosure is submitted. The basic invention is practised as follows:

The reactant used in the process of this invention comprises a hydroxyamine.

The catalyst comprises a transition metal containing an alkaline earth metal oxide.

As mentioned above catalysts which are suitable in the practice of this invention contain compounds of transition metals of the group consisting of copper, nickel, palladium or chromium. The transition metals can be chosen from a wide variety of organic or inorganic compounds, complexes, etc. It is only necessary that the catalyst precursor actually employed contain said metal in any of its ionic states. The actual catalytically active species may then comprise, for example copper, nickel or chromium or a combination thereof.

The copper-catalyst precursor may take many different forms. For instance the copper may be added to the catalyst precursor mix in an oxide, salt or complex form. For example, the copper may be introduced as cupric oxide, cuprous oxide, copper nitrate, copper sulfate, copper chloride, copper acetate and copper phosphate. Alternatively, the copper may be added as a complex derivative, such as copper(II) 2,4-pentanedionate, copper(II) gluconate and copper methoxide.

The nickel-catalyst precursor may also take many different forms. For instance the nickel may be added to the catalyst formulation in an oxide, salt or complex form. For example the nickel may be introduced as nickel oxide, nickel nitrate, nickel sulfate, nickel chloride and nickel acetate. Alternatively, the nickel may be added as a complex derivative such as nickel 2-ethylhexanoate, nickel(II) 2,4-pentanedionate and nickel glycinate.

The chromium-catalyst precursor may also take several different forms, but preferably it is added as the chromite or chromate ions. Particularly preferable is the addition of chromium as the chromite noiety in copper chromate. Chromium may also be added in an oxide, salt or complex form, such as for example, chromium nitrate, chromium(III) acetate, chromium(III) chloride and chromium phosphate, as well as sodium chromate.

Cadmium may likewise be added in many different forms. Palladium may be used on an inert support such as carbon.

In many cases the transition metal catalyst compounds are preferably employed with an oxide of an alkaline earth metal. Suitable components used in the preferred catalyst formulation may contain an oxide of barium, strontium, calcium, magnesium or beryllium.

Specific examples of such alkaline earth metal oxides include calcium oxide, strontium oxide and barium oxide, among others.

In the preferred embodiment of this invention the catalyst comprises barium oxide used in combination with a transition metal selected from the group consisting of copper, nickel and chromium. The preferred catalyst includes a transition metal salt of a mineral acid and barium oxide. Although compounds of each type which will work have been listed the following comprise some of the preferred combinations:

1) A copper chromate catalyst stabilized with barium oxide. Suitable examples include Harshaw catalysts Cu-0401, Cu-0402, Cu-1107, Cu-1184 and Cu-1230.

2) A copper chromium catalyst without stabilizing alkaline earth metal oxide added. Suitable examples include Harshaw catalyst Cu-1800P and Girdler T-1418.

3) A copper chromium catalyt with added cadmium examples include Girdler catalyst T-988.

4) Palladium-on-carbon catalysts.

5) Nickel, copper and chromium bulk metal catalysts such as, for example, Harshaw Ni-2715.

As mentioned above the preferred reactants are hydroxyamines. The hydroxyamines which will work in the process can be represented by the following structural formula:

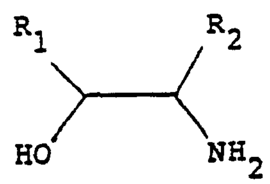

wherein $R_1$ is hydrogen or a hydrocarbon group having one to about 10 carbon atoms, such as an alkyl, aryl, aralkyl, alkaryl, alkenyl or cycloalkenyl group and $R_2$ is hydrogen or of the class of $R_1$. Hydroxyamines represented by this structure include Beta-hydroxyamines or 1,2-hydroxyamines.

Examples of suitable hydroxyamines include, but are not limited to ethanolamine, 1-amino-2-propanol, 1-amino-2-butanol, 1-amino-2-hexanol and 2-amino-3-butanol. Good results were obtained using 1-amino-2-propanol.

Said hydroxyamine reactants may be fed to the reaction mixture neat, or in the presence of an inert diluent. Water is the preferred diluent in the practice of this invention.

The quantity of transition-metal containing catalyst employed in the instant process is not critical and may vary over a wide range. Generally the reaction proceeds when employing as little as about $1 \times 10^{-6}$ weight percent and even lesser amounts of transition metal. The upper concentration is dictated by a variety of factors, including catalyst cost, operating temperature, etc.

The temperature range which can usefully be employed in these syntheses is a variable dependent upon other experimental factors, including the pressure, the concentration and the choice of the particular species of transition metal catalyst, among other things. The range of operability is from about 50° to 400° C. ° A narrow range of 150° -350° C represents the preferred temperature range.

Pressures of atmospheric or greater lead to substantial yields of pyrazine products by the process of this invention. A preferred operating range is above atmospheric pressure. The most preferred range is from 0-800 psi, but pressures greater than 800 psi can be used.

The novel process of this invention can be conducted in a batch, semi-continuous or continuous fashion and said material may be recovered by methods well known in the art, such as distillation, fractionation, extraction and the like.

The major products of this syntheses are pyrazines, including, but not limited to 2,5-dimethylpyrazine, pyrazine 2,5-dimethyl-3-propylpyrazine; 2,5-dimethyl-3-ethylpyrazine; and 2,3,5-trimethylpyrazine. The principal by-products of these preparations are piperazines but trialkylated pyrazines are also detected in some cases.

Yield, as defined herein, represents the efficiency in catalyzing the desired reaction relative to other undesired reactions. In this instance synthesis of pyrazines is the desired conversion. Yield is expressed as a molar percentile and is calculated by determining the molar amount of, for example, 2,5-dimethylpyrazine formed, divided by the molar amount of hydroxyamine charged and multiplying the quotient.

The products have been identified in this work by one or more of the following analytical procedures, viz, gas-liquid phase chromatography (glc), infrared (ir), mass spectrometry, proton nuclear magnetic resonance ($H^{\prime}$-nmr) and elemental analyses, or a combination of these techniques. All temperatures are in degrees centigrade and all pressures are in pounds per square inch gauge (psig).

Having described the inventive process, the following examples are submitted to supply specific and illustrative embodiments.

## SPECIFIC EXAMPLE 1

A 300 ml capacity stirred autoclave was charged with a mixture of 1-amino-2-propanol (40g, 0.53 mol) and 5g of Harshaw catalyst Cu-0401 (containing copper, chromium and barium). The reactor was sealed and purged of air. The reactor was then heated to 210° C with stirring, and held at temperature for one hour then allowed to cool. Maximum reactor pressure was 775 psig.

The light-green crude liquid product (39g) was weighed and analyzed.

The results showed:

| | |
|---|---|
| 1-amino-2-propanol conversion | 46.2 mol% |
| 2,5-dimethylpyrazine yield | 36.3 mol% |
| 2,5-dimethylpiperazine yield | 2.6 mol% |

## SPECIFIC EXAMPLES 2-6

Following the procedures of Example 1, 2,5-dimethylpyrazine was prepared from 1-amino-2-propanol using the following catalysts. Results and experimental conditions are summarized in Table I:

TABLE I

| Exp. | Catalysts | | Ratio of Catalyst To Hydroxyamine (W/W) | Yield, % | | | | | |
|---|---|---|---|---|---|---|---|---|---|
| | Name | Major Metals Present | | Temp. °C | Time, Hr | Max. Press., psi | Conversion, % | 2,5-dimethyl-pyrazine | 2,5-dimethylpiperazine |
| 2 | Hashaw Ni-2715 | Ni-Cu-Cr | 0.5 | 193 | 4 | 300 | 100 | 19.5 | 53.2 |
| 3 | Harshaw Cu-1800P | Cu-Cr | 0.1 | 210 | 0.5 | 780 | 66.5 | 35.0 | 12.1 |
| 4 | Girdler T-988 | Cu-Cr-Cd | 0.125 | 150 | 10 | 100 | 15.3 | 1.7 | 0.3 |
| 5 | Matthey Bishop | Pd/C | 0.005 | 180 | 5 | 340 | 34.8 | 16.6 | 5.8 |
| 6 | Girdler T-1418 | Cu-Cr | 0.25 | 195 | 2 | 650 | 76.7 | 32.1 | 14.5 |

## SPECIFIC EXAMPLE 7

A tubular reactor was filled with 50 cc of Harshaw Cu-0402 catalyst. The liquid feed (1-amino-1-propanol) was introduced upflow at the rate of 12 cc per hour under atmospheric pressure and the reactor heated to temperature. The products were collected after the reaction was prerun for two hours at each temperature. The results are listed in Table II in which the yield and conversion are based on the hydroxyamine used.

TABLE II

| Temp., °C | Conversion, % | Yield, % | |
|---|---|---|---|
| | | 2,5-dimethylpyrazine | 2,5-dimethylpiperazine |
| 190 | 8.7 | 5.1 | 0 |
| 200 | 13.8 | 9.5 | Trace |
| 210 | 17.4 | 13.0 | Trace |
| 221 | 22.7 | 17.5 | 0 |
| 230 | 28.7 | 23.4 | Trace |
| 241 | 37.2 | 32.1 | Trace |
| 250 | 45.1 | 39.2 | Trace |
| 261 | 57.4 | 50.7 | Trace |
| 271 | 71.6 | 64.5 | 0.2 |
| 280 | 83.0 | 77.2 | 0.4 |
| 290 | 93.8 | 84.6 | 0.7 |
| 300 | 99.9 | 89.9 | 1.3 |
| 310 | 99.4 | 89.5 | 0.4 |

## SPECIFIC EXAMPLE 8

Ethanolamine was subjected to a reaction as described in Example 7 and the reactor temperature was at 300° C. Pyrazine was obtained as the major cyclocondensation product.

## SPECIFIC EXAMPLE 9

A tubular reactor was filled with 50 cc of Harshaw Cu-1107 catalyst (containing Cu-Cr-Ba). The feed (50% 1-amino-2-propanol in water) was introduced at the rate of 24 cc per hour under atmosphere at 300° C. The products were collected after the reaction was prerun for two hours. A 70% yield of 2,5-dimethylpyrazine was obtained with 81% conversion of 1-amino-2-propanol.

## SPECIFIC EXAMPLE 10

Harshaw Cu-1184 catalyst (containing Cu-Cr-Ba) was subjected to a reaction as described in Example 9 except that the reaction temperature was 270° C. An 87% yield of 2,5-dimethylpyrazine was obtained with 99% conversion of 1-amino-2-propanol.

SPECIFIC EXAMPLE 11

Harshaw Cu-1230 catalyst (containing Cu-Cr-Ba) was subjected to a reaction as described in Example 9 except that the reaction temperature was 270° C. A 85% yield of 2,5-dimethylpyrazine was obtained with 99% conversion of 1-amino-2-propanol.

SPECIFIC EXAMPLE 12

The 1-amino-2-butanol was subjected to a reaction as described in Example 3. A 38% yield of 2,5-diethylpyrazine and 21% yield of 2,5-diethylpiperazine were obtained with an 87% conversion of 1-amino-2-butanol.

## Claims

1. A process for preparing pyrazines characterised in that it comprises cyclocondensation of a hydroxyamine in the presence of a catalyst comprising a rhodium, ruthenium or palladium-containing compound and a phosphine containing compound or in the presence of a catalyst containing one or more transition metals selected from copper, nickel, chromium and palladium, the reaction being carried out at elevated temperature and at atmospheric pressure or greater.

2. A process according to claim 1 characterised in that the catalyst is a rhodium, ruthenium or palladium-containing compound and a phosphine containing compound and that the reaction is carried out at a temperature of at least 100° C.

3. A process according to claim 2 characterised in that the hydroxyamine is of the formula:

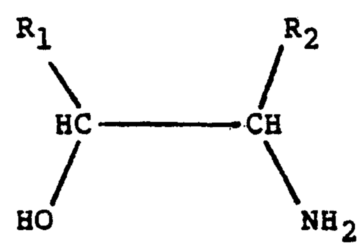

wherein $R_1$ is a hydrocarbon group having one to about 6 carbon atoms consisting of alkyl, alkenyl or cycloalkyl group and $R_2$ is hydrogen or of the class of $R_1$.

4. A process according to any of claims 2, 3 and 4 characterised in that the hydroxyamine is selected from ethanolamine, 1-amino-2-butanol, 1-amino-3-butanol and 1-amino-2-propanol.

5. A process according to any of claims 2 to 5 characterised in that the rhodium-containing compound and the ruthenium compound are the respective salts of a mineral acid

6. A process according to claim 5 characterised in that the rhodium salt of a mineral acid is rhodium (III) chloride and that the ruthenium salt of a mineral acid is ruthenium (III) chloride.

7. A process according to any of claims 2 to 6 characterised in that the phosphine-containing compound is selected from tri-n-butylphosphine, triphenylphosphine and 1,3-bis(diphenyl-phosphino)-propane.

8. A process according to any of claims 2 to 8 characterised in that the rhodium, ruthenium or palladium-containing compound and the phosphine-containing compound are added as a preformed complex.

9. A process according to claim 8 characterised in that said complexes are selected from tris-(triphenylphosphine)ruthenium(II) chloride, bis(triphenylphosphine)palladium(II) chloride and tris-(triphenylphosphine)(carbonyl)rhodium(I) hydride.

10. A process according to any of claims 2 to 9 characterised in that the rhodium, ruthenium, or palladium-containing compound and the phosphine-containing compound are selected from the following combinations: $RuCl_3$-$Bu_3P$, $RuCl_3$-$Ph_2P(CH_2)_3PPh_2$ and $RhCl_3$-$Ph_2P(CH_2)_3PPh_2$.

11. A process according to any of claims 2 to 10 characterised in that the cyclocondensation is

conducted in the presence of an inert solvent.

12. A process according to claim 11 characterised in that the said solvent is selected from cyclic, acyclic and heterocyclic ethers.

13. A process according to claim 12 characterised in that the solvent is selected from p-dioxane and tetraglyme.

14. A process according to any of claims 2 to 13 characterised in that the temperature is 150° C to 250° C.

15. A process according to any of claims 2 to 14 characterised in that the pressure is from 100 to about 400 psi.

16. A process according to claim 1 characterised in that the catalyst is the heterogeneous catalyst containing one or more transition metals selected from copper, nickel, chromium and palladium and that the reaction is carried out at at least 50° C.

17. A process according to any of claims 1 to 16 characterised in that the hydroxyamine is of the formula:

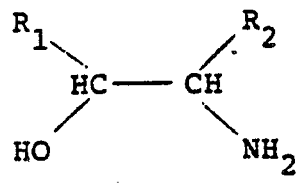

wherein $R_1$ is a hydrocarbon group having 1 to about 10 carbon atoms selected from the group consisting of alkyl, aryl, aralkyl, alkaryl, alkenyl or cycloalkyl group and $R_2$ is hydrogen or of the class of $R_1$.

18. A process according to any of claims 16 and 17 characterised in that the hydroxyamine is selected from ethanol amine, 2-amino-3-butanol, 1-amino-2-butanol and 1-amino-2-propanol.

19. A process according to any of claims 16 to 18 characterised in that the metal catalyst contains a copper and chromium-containing compound selected from Harshaw Cu-1800; Harshaw Cu-0401; Harshaw Cu-0402; Harshaw Cu-1107; Cu-1184 and Cu-1230.

20. A process according to claim 19 characterised in that it comprises a copper-nickel-chromium-containing catalyst which is Harshaw catalyst Ni-2715.

21. A process according to any of claims 16 to 18 characterised in that the catalyst is palladium-on-carbon.

22. A process according to any of claims 16 to 21 characterised in that the transition metal catalyst contains an alkaline earth metal oxide promoter.

23. A process according to claim 22 characterised in that the alkaline earth metal oxide promoter is selected from the oxides of barium, strontium, calcium and magnesium.

24. A process according to any of claims 16 to 23 characterised in that the transition metal compound and alkaline earth metal oxide are combined.

25. A process according to any of claims 16 to 24 characterised in that the temperature is 150° C to 350° C.

26. A process according to any of claims 16 to 25 characterised in that the pressure is from 0 to about 800 psi.

European Patent Office

# EUROPEAN SEARCH REPORT

Application Number

EP 88 30 9456

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| Y | JOURNAL OF ORGANIC CHEMISTRY, vol. 50, 1985, pages 1365-1370, American Chemical Society, Washington, D.C., US; Y. TSUJI et al.: "Ruthenium complex catalyzed N-heterocyclization. Syntheses of N-substituted piperidines, morpholines, and piperazines from amines and 1,5-diols" * Pages 1365-1366,1368; page 1369, column 2 * | 1,2,6-15 | C 07 D 241/12<br>B 01 J 31/00<br>B 01 J 23/00 |
| X,Y | US-A-3 067 199 (W.K. LANGDON) * Column 1, lines 3-7; columns 9,10,14,26 * | 1,3,16-18,25,26 | |
| X | FR-A-2 150 092 (VEBA-CHEMIE) * Pages 1,3,5-8 * | 1,3,16-18,25,26 | |
| D,A | PATENT ABSTRACTS OF JAPAN, vol. 4, no. 182 (C-35)[664], 16th December 1980; & JP-A-55 122 769 (MITSUWAKA JIYUNYAKU KENKYUSHO K.K.) 20-09-1980 * Abstract * | 1,3,16,25,26 | TECHNICAL FIELDS SEARCHED (Int. Cl.3)<br>C 07 D 241/00 |

The present search report has been drawn up for all claims

| Place of search | Date of completion of the search | Examiner |
|---|---|---|
| THE HAGUE | 12-06-1989 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document